# EUROPEAN PATENT APPLICATION

(11) **EP 2 727 576 A1**
(43) Date of publication of application: **07.05.2014**
(21) Application number: 12007522.1
(22) Date of filing: 05.11.2012
(51) Int. Cl.: A61K 6/083, C07D 307/14

(54) **Dental composition**

(71) Applicant: DENTSPLY DETREY GmbH, 78467 Konstanz (DE)
(72) Inventor: Klee, Joachim, E. Prof. Dr., 78315 Radolfzell (DE); Elsner, Oliver Dr., 78476 Allensbach (DE); Fik, Christoph P., 78256 Steißlingen (DE); Gansel, Julia Dr., 78315 Radolfzell (DE); Lampe, Ulrich, 40225 Düsseldorf (DE); Maier, Maximillian, 40213 Düsseldorf (DE); Ritter, Helmut Prof. Dr., 42111 Wuppertal (DE); Kavallar, Lisa-Maria, 46049 Oberhausen (DE)
(74) Representative: Hartz, Nikolai

(57) **Abstract**

Dental composition comprising a polymerizable compound of the following formula (I):

A-L(B)ₙ (I)

wherein A, L, B and n are as defined in claim 1.

## Description

### Field of the Invention

The present invention relates to a dental composition comprising a specific polymerizable compound. Furthermore, the present invention relates to the use of the specific polymerizable compound for the preparation of a dental composition. The polymerizable compound of the present invention is a water soluble yet hydrolysis-stable polyfunctional polymerizable monomer, which is copolymerizable with conventional (meth)acrylates and which provides compositions having a viscosity which may be adjusted to a low range, and which has biocompatibility.

### Background of the Invention

Dental compositions containing polymerizable compounds are known. Conventionally, polymerizable dental compositions are provided for a broad range of applications and must, therefore, meet diverse requirements. For example, a polymerizable dental composition may be a dental adhesive composition, a bonding agent, a pit and fissure sealant, a dental desensitizing composition, a pulp capping composition, a dental composite, dental glass ionomer cement, a dental cement, resin modified glass ionomers, or a dental root canal sealer composition.

Conventionally, (meth)acrylates are used as polymerizable components in polymerizable dental compositions due to their excellent reactivity in radical polymerizations which may be demonstrated based on the polymerization enthalpy, i.e. Δ_{R}H = -58 kJ/mol per double bond in case of methacrylates and Δ_{R}H = -80 kJ/mol per double bond in case of acrylates. Based on a typical degree of polymerization in a photo polymerization of about 70 %, a Δ_{R}H = -41 kJ/mol per double bond in case of methacrylates and a Δ_{R}H = -58 kJ/mol per double bond in case of acrylates is frequently observed in case of a dental composition.

In order to provide crosslinking capability, polyfunctional (meth)acrylates such as bis-GMA, were used for dental applications as early as 1962.

However, hydrolysis stability of (meth)acrylates is problematic in view of the acidity of many dental compositions which severely limits the storage stability of the dental composition. EP1234543 discloses (meth)acrylamides having an increased hydrolysis stability. However, certain (meth)acrylamides have an increased viscosity as compared to the corresponding (meth)acrylates which is undesireable in the case of dental compositions wherein the polymerizable matrix is required to have a low viscosity.

### Summary of the Invention

It is the problem of the present invention to provide a dental composition comprising a hydrolysis stable polyfunctional polymerizable monomer, which is copolymerizable with conventional (meth)acrylates and which has a low viscosity and biocompatibility.

The present invention provides a composition comprising a polymerizable compound of the following formula (I):

A-L(B)ₙ (I)

- A: is a group of the following formula (II) 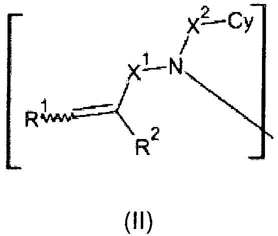
wherein
- X¹: is CO or CS;
- X²: is a single bond, CO, CS, CH₂, or a group Z[X'Z']ₖ, wherein X' is an oxygen atom, a sulfur atom or NR, (wherein R is a hydrogen atom, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or -SO₃M), Z is a straight chain or branched C₁₋₄ alkylene group, Z' is a bond, a straight chain or branched C₁₋₄ alkylene group, and k is an integer of from 1 to 50;
- Cy: is a monocylic, bicyclic or tricyclic cycloalkyl group having 3 to 20 carbon atoms or a monocylic, bicyclic or tricyclic heterocycloalkyl group having 2 to 20 carbon atoms and 1 to 4 heteroatoms selected from oxygen, sulfur and nitrogen atoms, which groups may be substituted with 1 to 3 groups selected from a C₁₋₁₆ alkyl group, - COOM, -PO₃M, -O-PO₃M₂ or -SO₃M;
- R¹: is a hydrogen atom,
-COOM,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M,
a C₆₋₁₄ aryl or C₃₋₁₄ heteroaryl group which may be substituted by -COOM, -PO₃M, - O-PO₃M₂ or -SO₃M,
- R²: is a hydrogen atom,
-COOM,
a straight chain or branched C₁₋₁₆ alkyl group which may be substituted by a C₆₋₁₄ aryl or C₃₋₁₄ heteroaryl group, -COOM, -PO₃M, -O-PO₃M₂ and -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, or
a C₆₋₁₄ aryl or C₃₋₁₄ heteroaryl group which may be substituted by -COOM, -PO₃M, - O-PO₃M₂ and -SO₃M,
- L: is a single bond or an (n+1) -valent linker group;
- B: independently is
a group according to the definition of A,
a group of the following formula (III) 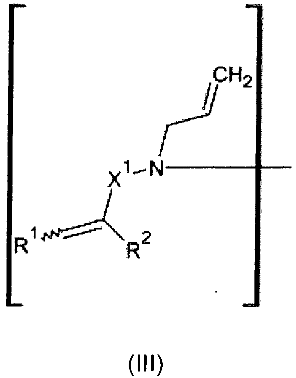 wherein
- X¹: independently has the same meaning as defined for X¹ in formula (II), CH₂, or a group [X'Z]_{k'}, wherein X' is an oxygen atom, a sulfur atom or NH, Z is a straight chain or branched C₁₋₄ alkylene group, and k' is an integer of from 1 to 50;
- R¹ and R²: are independent from each other and independently have the same meaning as defined for formula (II),
a group of the following formula (IV) 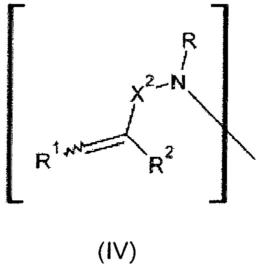 wherein
- X²: independently has the same meaning as defined for X¹ in formula (III),
- R¹ and R²: are independent from each other and independently have the same meaning as defined for formula (II),
- R: is a hydrogen atom,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -0-PO₃M₂ or -SO₃M,
a C₆₋₁₄ aryl or C₃₋₁₄ heteroaryl group which may be substituted by - COOM, -PO₃M, -O-PO₃M₂ or -SO₃M,
a group of the following formula (V) 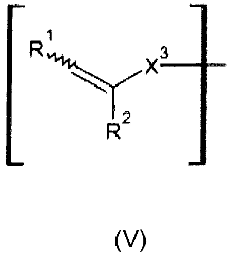 wherein
X³ is CH₂, CO, -CH₂CO-, CS, or -CH₂CS-,
R¹ and R² which are independent from each other and independently have
the same meaning as defined for formula (II), or
a group [Z'X"]ₘE,
wherein
- Z': is a straight chain or branched C₁₋₄ alkylene group,
- X": is an oxygen atom, a sulfur atom or NR, (wherein R is a hydrogen atom, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, - PO_{3M}, -O-PO₃M₂ or -SO₃M),
- E: is a hydrogen atom,
PO₃M₂,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -0-PO₃M₂ or -SO₃M,
a C₆₋₁₄ aryl or C₃₋₁₄ heteroaryl group which may be substituted by - COOM, -PO₃M, -O-PO₃M₂ or -SO₃M, and m is an integer of from 1 to 10; and

- n: is an integer of from from 1 to 6;
wherein M which are independent from each other each represent a hydrogen atom or a metal atom;
provided that in case L is a single bond, B cannot be a group according to the definition of A or a group of the formula (III) or (IV).

The present invention also provides a use of a compound of formula (I) for the preparation of a dental composition selected from a dental adhesive composition, a bonding agent, a pit and fissure sealant, a dental desensitizing composition, a pulp capping composition, a dental composite, dental glass ionomer cement, a resin modified dental cement, a dental cement and a dental root canal sealer composition.

The present invention is based on the recognition that the viscosity of the compounds of formula (I) is comparably low as compared to the corresponding (meth)acrylamides.

### Detailled Description of Preferred Embodiments

In this description, unless otherwise specified, a halogen atom denotes a fluorine atom, a chlorine atom, a bromine atom or a iodine atom; an alkyl group denotes, for example, a straight-chain or branched-chain C₁₋₁₆ alkyl group such as methyl, ethyl, propyl, isopropyl, butyl, sec-butyl, isobutyl, tert-butyl, pentyl, hexyl and octyl; a cycloalkyl group denotes a C₃₋₆ cycloalkyl group such as cyclopropyl, cyclobutyl, cyclopentyl and cyclohexyl; an aryl group denotes a C₆₋₁₄ aryl group such as phenyl, naphthyl; a heteroaryl group denotes a C₃₋₁₄ heteroaryl group which may contain 1 to 3 heteroatoms selected from nitrogen, oxygen and sulfur, such as furyl, thienyl, pyrrolyl, imidazolyl, pyrazolyl, thiazolyl, isothiazolyl, oxazolyl, isoxazolyl, thiadiazolyl, furazanyl, pyrrolidinyl, imidazolidinyl, pyridyl, pyrimidinyl, pyridzinyl, pyrazinyl, piperazinyl, piperidyl, morpholinyl, thiomorpholinyl, benzofuranyl, isobenzofuranyl, benzothienyl, indolinyl, isoindolinyl, indolizinyl, benzimidazolyl, benzoxazolyl, benzisoxazolyl, benzothiazolyl, benzoisothiazolyl, purinyl, coumarinyl, phthalazinyl, naphthyridinyl, quinoxalinyl, quinazolinyl, cinnolinyl, chromanyl, isochromanyl, chromenyl, thiochromenyl, quinuclidinyl, 1,3-benzodioxolyl, benzothiadiazolyl, benzoxadiazolyl, benzodioxanyl, quinolyl, isoquinolyl; an alkylene group denotes a C₁₋₄ alkylene group such as methylene, ethylene, propylene, butylene and hexylene.

The present invention provides a dental composition. The dental composition of the present invention is polymerizable or copolymerizable by a radical polymerization. The dental composition may be selected from a dental adhesive composition, a bonding agent, a pit and fissure sealant, a dental desensitizing composition, a pulp capping composition, a dental composite, dental glass ionomer cement, a resin modified dental cement, a dental cement and a dental root canal sealer composition.

A dental composition according to the present invention comprises a polymerizable compound of the following formula (I). The dental composition may comprise one or more compounds of formula (I). The dental composition of the present invention may comprise the polymerizable compound in an amount of from 0.2 to 99 percent by weight based on the total weight of the composition. Preferably, a dental adhesive composition comprises 0.5 to 50 percent by weight of one or more compounds of formula (I). Preferably, a bonding agent comprises 0.5 to 70 percent by weight of one or more compounds of formula (I). Preferably, a pit and fissure sealant comprises 0.5 to 80 percent by weight of one or more compounds of formula (I). Preferably, a dental desensitizing composition comprises 0.5 to 90 percent by weight of one or more compounds of formula (I). Preferably, a pulp capping composition comprises 0.5 to 50 percent by weight of one or more compounds of formula (I). Preferably, a dental composite comprises 0.5 to 50 percent by weight of one or more compounds of formula (I). Preferably, a dental glass ionomer cement comprises 0.5 to 50 percent by weight of one or more compounds of formula (I). Preferably, a resin modified dental cement comprises 0.5 to 50 percent by weight of one or more compounds of formula (I). Preferably, a dental cement comprises 0.5 to 50 percent by weight of one or more compounds of formula (I).

A compound of formula (I) is according to the following formula (I):

A-L(B)ₙ (I)

In formula (I), A is a specific polymerizable group which is linked by a single bond to a single group B or by a linking group to up to six groups B, preferably up to four groups B. In case more than one group B is present in the polymerizable compound of formula (I), the groups B may be the same or different.

In order to avoid a nitrogen-nitrogen single bond, B cannot be a group according to the definition of A or a group of the following formula (III) in case L is a single bond.

According to the present invention, A is a group of the following formula (II)

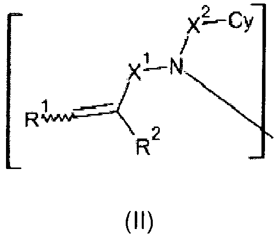

Accordingly, any compound of formula (I) is characterized by a cyclic group bonded to a nitrogen atom and a further specific group having a polymerizable double bond which is bonded to the same nitrogen atom.

In formula (II), X¹ is CO, CS. According to a preferred embodiment, X¹is CO.

In formula (II), X² is a single bond, CO, CS, CH₂, or a group Z[X'Z']ₖ, wherein X' is an oxygen atom, a sulfur atom or NR, (wherein R is a hydrogen atom, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, - COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or -SO₃M), Z is a straight chain or branched C₁₋₄ alkylene group, and k is an integer of from 1 to 50, and Z' is a bond, a straight chain or branched C₁₋₄ alkylene group, and k is an integer of from 1 to 50, preferably of from 2 to 10.

In formula (II), R¹ is a hydrogen atom, -COOM, a straight chain or branched C₁₋₁₆ alkyl group which may be substituted by a C₃₋₆ cycloalkyl group, a C₆₋₁₄ aryl or C₃₋₁₄ heteroaryl group, - COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or - SO₃M, a C₆₋₁₄ aryl or C₃₋₁₄ heteroaryl group which may be substituted by -COOM, -PO₃M, - O-PO₃M₂ or -SO₃M.

In formula (II), R¹ may be a straight chain or branched C₁₋₁₆ alkyl group, preferably a straight chain or branched C₁₋₆ alkyl group. Examples of the C₁₋₁₆ alkyl group of R¹ methyl, ethyl, n-propyl, i-propyl, n-butyl, isobutyl, tert-butyl sec-butyl, pentyl and hexyl. An aryl group may, for example, be a phenyl group or a naphthyl group. A C₃₋₁₄ heteroaryl group may contain 1 to 3 heteroatoms selected from nitrogen, oxygen and sulfur.

According to a preferred embodiment, R¹ is a hydrogen atom. In case R¹ is a C₁₋₆ alkyl group, the C₁₋₆ alkyl group is preferably substituted by -COOM.

In formula (II), R² is a hydrogen atom, -COOM, a straight chain or branched C₁₋₁₆ alkyl group which may be substituted by a C₆₋₁₄ aryl or C₃₋₁₄ heteroaryl group, -COOM, -PO₃M, -0-PO₃M₂ and -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, or a C₆₋₁₄ aryl or C₃₋₁₄ heteroaryl group which may be substituted by -COOM, -PO₃M, -O-PO₃M₂ and -SO₃M.

In formula (II), R² may be a straight chain or branched C₁₋₁₆ alkyl group, preferably a straight chain or branched C₁₋₆ alkyl group. Examples of the C₁₋₁₆ alkyl group of R² methyl, ethyl, n-propyl, i-propyl, n-butyl, isobutyl, tert-butyl sec-butyl, pentyl and hexyl. An aryl group may, for example, be a phenyl group or a naphthyl group. A C₃₋₁₄ heteroaryl group may contain 1 to 3 heteroatoms selected from nitrogen, oxygen and sulfur.

According to a preferred embodiment, R² is a hydrogen atom or a methyl group.

In formula (II), Cy is a monocylic, bicyclic or tricyclic cycloalkyl group having 3 to 20 carbon atoms or a monocylic, bicyclic or tricyclic heterocycloalkyl group having 2 to 20 carbon atoms and 1 to 4 heteroatoms selected from oxygen, sulfur and nitrogen atoms, which groups may be substituted with 1 to 3 groups selected from a C₁₋₁₆ alkyl group, -COOM, - PO₃M, -O-PO₃M₂ or -SO₃M. According to a preferred embodiment, Cy is a monocyli or bicyclic cycloalkyl group having 3 to 10 carbon atoms or a monocylic or bicyclic group having 2 to 10 carbon atoms and 1 to 4 heteroatoms selected from oxygen, sulfur and nitrogen atoms.

According to a preferred embodiment, Cy may be selected from the following groups:

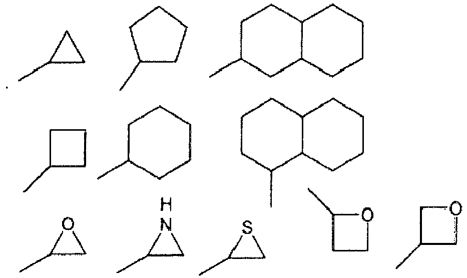

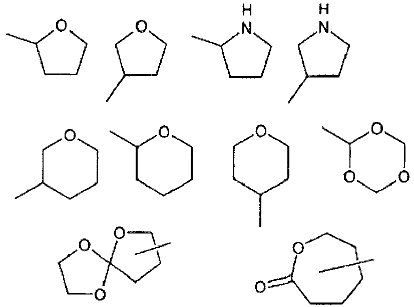

The following groups are preferred groups of formula (II), wherein M is a hydrogen atom or a metal atom:

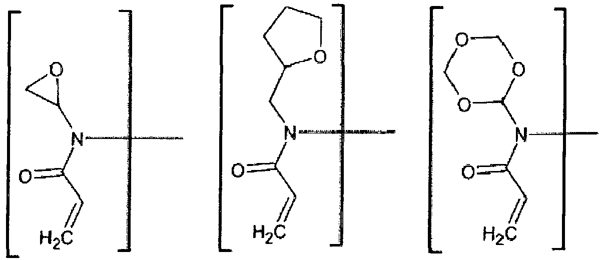

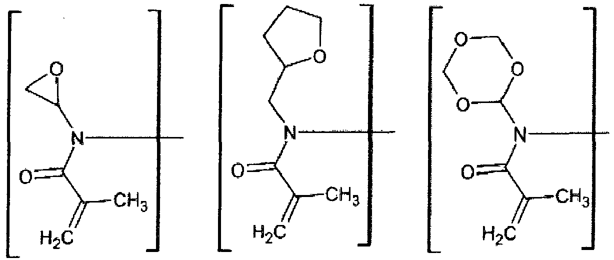

In formula (I), L is a single bond or an (n+1) -valent linker group.

The linker group has a valency of at least two which corresponds to the total number of substituents A and B. Accordingly, L may be preferably divalent (n=1), trivalent (n=2), tetravalent (n=3), or pentavalent (n=4). Preferable, when L is a linker group, L is divalent or trivalent, most preferably divalent.

In general, when L is a linker group, L is a linear or branched monomeric, oligomeric, polymeric or copolymeric group. A monomeric groups is a low-molecular group having a molecular weight of up to 500. An oligomeric group is a group having a molecular weight of more than 500 to up to 10000 and may be prepared by a polymerization reaction. A polymeric or copolymeric group is a group having a molecular weight of more than 10000 which may be obtained by a polymerization reaction.

The linker group L may be a hydrocarbon group which may be aliphatic and/or aromatic. The hydrocarbon group may be substituted by 1 to 6 C₁₋₄ alkyl groups. Specific examples of the alkyl groups are methyl, ethyl, n-propyl, i-propyl, n-butyl, i-butyl or tert.-butyl. In a preferred embodiment, the hydrocarbon group of the linker group L may contain 1 to 5 oxygen atoms in the hydrocarbon group in the form of aliphatic or aromatic ether bonds, keto groups, carboxylic acid groups, or hydroxyl groups. Ester groups are not preferred in moiety L in view of hydrolysis stability of the polymerizable monomer. In case of an aliphatic group, L may be a straight chain or branched chain alkylene group or a cycloalkylene group. In case of an aromatic group, A may be an arylene group or a C₃₋₁₄ heteroarylene group. Specifically, L may be a divalent substituted or unsubstituted C₁ to C₂₀ alkylene group, substituted or unsubstituted C₆₋₁₄ arylene group, substituted or unsubstituted C₃ to C₂₀ cycloalkylene group, substituted or unsubstituted C₇ to C₂₀ arylenealkylenearylene group.

According to a preferred embodiment, L represents a saturated aliphatic C₂₋₂₀ hydrocarbon chain which may contain 2 to 4 oxygen atoms or nitrogen atoms, and which may be substituted by 1 to 6 C₁₋₄ alkyl groups, or L may be a substituted or unsubstituted C₇ to C₂₀ arylenealkylenearylene group which may be substituted by 1 to 6 C₁₋₄ alkyl groups.

In case L is an (n+1) -valent linker group, the linker group may preferably be a C₁₋₁₂ hydrocarbon group. The C₁₋₁₂ hydrocarbon group may contain 1 to 3 carbonyl groups or heteroatoms selected from oxygen, nitrogen and sulfur. Moreover, the C₁₋₁₂ hydrocarbon group may be substituted by a hydroxyl group, a C₆₋₁₄ aryl group, -COOM, -PO₃M, -0-PO₃M₂ or -SO₃M, wherein M is a hydrogen atom or a metal atom. Specific examples of a divalent C₁₋₁₂ hydrocarbon group are a straight chain or branched C₁₋₁₂ alkylene group such as a methylene, ethylene, propylene, butylene or penylene group which groups may be substituted by a hydroxyl group, a C₆₋₁₄ aryl group, -COOM, -PO₃M, -O-PO₃M₂ or -SO₃M.

In case L is an divalent linker group, the linker group may be polyamide residue of the following formula (VI)

-Y-L'-Y'- (VI)

wherein
Y and Y' represent CO and the polyamide moiety of formula (V) is obtainable by a process comprising the step of a step-growth polymerization of a mixture containing a compound having at least two amino groups of the following formula (VII) and a compound of the following formula (VIII) having at least two carboxylic acid groups,

R³(NHR")_{y} (VII)

wherein
- R³: represents an y-valent C₂₋₂₀ straight-chain, branched or cyclic hydrocarbon group which may optionally contain from 1 to 6 hetero atoms selected from nitrogen, oxygen, or sulphur atoms in the backbone of the hydrocarbon group, and optionally from 1 to 6 functional groups selected from hydroxyl groups, thiol groups and amino groups;
- R": represents a hydrogen atom, an allyl group,
a straight chain or branched C₁₋₆ alkyl group which may be substituted by a C₃₋₆ cycloalkyl group, or a C₆₋₁₄ aryl group,
a C₃₋₆ cycloalkyl group which may be substituted by a C₁₋₆ alkyl group or a C₆₋₁₄ aryl group; and
- y: represents an integer of at least 2;

MOOC-R⁴-COOM (VIII)
wherein R⁴ represents a C₁₋₂₀ straight-chain, branched, cyclic or aromatic hydrocarbon group which may optionally contain a carbon-carbon double bond, from 1 to 6 heteroatoms selected from nitrogen, oxygen, or sulphur atoms in the backbone of the hydrocarbon group, and optionally from 1 to 6 functional groups selected from carboxylic acid groups, hydroxyl groups, thiol groups and amino groups, and the M which may be the same or different, independently represent a hydrogen atom or a metal atom.

Preferred divalent linker groups may be selected from methylene, ethylene, propylene, butylene and the following divalent groups:

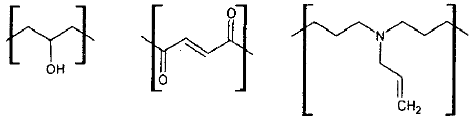

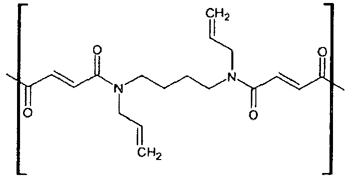

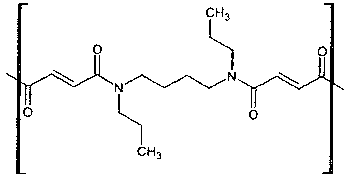

In formula (I), B independently is a group according to the definition of A, a group of the following formula (III), a group of the following formula (IV), a group of the following formula (V), or a group of the following formula [Z'X"]ₘE.

When B is a group according to formula (II) in the definition of A, then B may be the same or different from the group A present in a polymerizable compound of formula (I). Moreover, in case more than one group B according to the definition of A is present in the polymerizable compound of formula (I), the groups B may be the same or different. Specifically, B may be a group of the above formula (II) wherein an allyl group is bonded to a nitrogen atom, and a further group having a polymerizable double bond is bonded to the same nitrogen atom.

When B is a group according to formula (II) in the definition of A, X¹ is CO, CS, CH₂, or a group [X'Z]ₖ, where in X' is an oxygen atom, a sulfur atom or NH, Z is a straight chain or branched ₁₋₄ alkylene group, and k is an integer of from 1 to 10.

When B is a group according to formula (II) in the definition of A and in case X¹ is a group [X'Z]ₖ), it is preferred that L provides a polymerizable double bond which may take part with the allyl group directly bonded to the nitrogen atom of the group of formula (II) in a cyclopolymerization reaction. Preferably, X' is an oxygen atom. Preferred Examples for a straight chain or branched C₁₋₄ alkylene group for Z are an ethylene group and a propylene group. Preferably, k is an integer of from 1 to 4.

According to a preferred embodiment of B being a group according to formula (II) in the definition of A, X¹ is CO.

When B is a group according to formula (II) in the definition of A, R¹ is a hydrogen atom, - COOM, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or - SO₃M, a C₃₋₆ cycloalkyl group which may be substituted by a C₁₋₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or -SO₃M.

According to a preferred embodiment, R¹ is a hydrogen atom. In case R¹ is a C₁₋₆ alkyl group, the C₁₋₆ alkyl group is preferably substituted by -COOM.

When B is a group according to formula (II) in the definition of A, R² is a hydrogen atom, - COOM, a straight chain or branched C₁₋₁₆ alkyl group which may be substituted by a C₆₋₁₄ aryl group, -COOM, -PO₃M, -O-PO₃M₂ and -SO₃M, a C₃₋₆ cycloalkyl group which may be substituted by a C₁₋₁₆ alkyl group, a C₆₋₁₄ aryl group, -COOM, -PO₃M, -O-PO₃M₂ or -SO₃M, or a C₆₋₁₄ aryl or C₃₋₁₄ heteroaryl group which may be substituted by -COOM, -PO₃M, -0-PO₃M₂ and -SO₃M,

According to a preferred embodiment of B being a group according to formula (II) in the definition of A, R² is a hydrogen atom or a methyl group.

When B is a group of the formula (III), B is as follows: 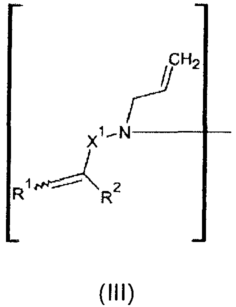 wherein X¹ independently has the same meaning as defined for X¹ in formula (II), CH₂, or a group [X'Z]_{k'}, wherein X' is an oxygen atom, a sulfur atom or NH, Z is a straight chain or branched C₁₋₄ alkylene group, and k' is an integer of from 1 to 50, preferably 2 to 10. R¹ and R² are independent from each other and independently have the same meaning as defined for formula (II).

When B is a group of the formula (IV), B is as follows.

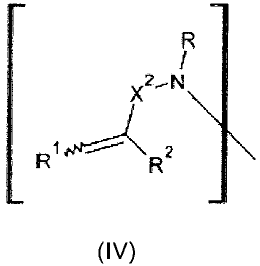

In formula (IV), X² has the same meaning as defined for X¹ in formula (II). Moreover, in case more than one group X² according to the definition of X¹ is present in the polymerizable compound of formula (I), the groups X² may be the same or different. Specifically, When B is a group according to formula (IV), X² is CO, CS, CH₂, or a group [X'Z]ₖ, wherein X' is an oxygen atom, a sulfur atom or NH, Z is a straight chain or branched C₁₋₄ alkylene group, and k is an integer of from 1 to 10. X', Z and m of a group of formula (III) maybe the same or different of X', Z and k of any group of formula (II) present as A or B in the polymerizable compound of fromula (I).

When B is a group according to formula (IV) and in case X² is a group [X'Z]ₖ), it is preferred that L provides a polymerizable double bond which may take part with the allyl group directly bonded to the nitrogen atom of the group of formula (II) in a cyclopolymerization reaction. Preferably, X' is an oxygen atom. Preferred Examples for a straight chain or branched C₁₋₄ alkylene group for Z are an ethylene group and a propylene group. Preferably, k is an integer of from 1 to 4.

According to a preferred embodiment of B being a group according to formula (IV), X¹ is CO.

In formula (IV), R¹ and R² are independent from each other and independently have the same meaning as defined for formula (II). Moreover, in case more than one group R¹ and R², respectively, is present when more than one group B of formula (III) is present in the polymerizable compound of formula (I), each of the groups R¹ and R² may be the same or different, respectively.

Specifically, when B is a group according to formula (IV), R¹ is a hydrogen atom, -COOM, a straight chain or branched C₁₋₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or -SO₃M.

According to a preferred embodiment when B is a group according to formula (IV), R¹ is a hydrogen atom. In case R¹ is a C₁₋₁₆ alkyl group, the C₁₋₆ alkyl group is preferably substituted by -COOM.

When B is a group according to formula (IV), R² is a hydrogen atom, -COOM, a straight chain or branched C₁₋₆ alkyl group which may be substituted by a C₆₋₁₄ aryl or C₃₋₁₄ heteroaryl group, -COOM, -PO₃M, -O-PO₃M₂ and -SO₃M, a C₃₋₆ cycloalkyl group which may be substituted by a C₁₋₆ alkyl group, a C₆₋₁₄ aryl or C₃₋₁₄ heteroaryl group, -COOM, - PO₃M, -O-PO₃M₂ or -SO₃M, or a C₆₋₁₄ aryl or C₃₋₁₄ heteroaryl group which may be substituted by -COOM, -PO₃M, -O-PO₃M₂ and -SO₃M,

According to a preferred embodiment of B being a group according to formula (IV), R² is a hydrogen atom or a methyl group.

In formula (IV), R is a hydrogen atom, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl group which may be substituted by -COOM, -PO₃M, -O-PO₃M₂ or -SO₃M.

Preferred groups of B of the formula (IV) are as follows wherein M is a hydrogen atom or a metal atom.

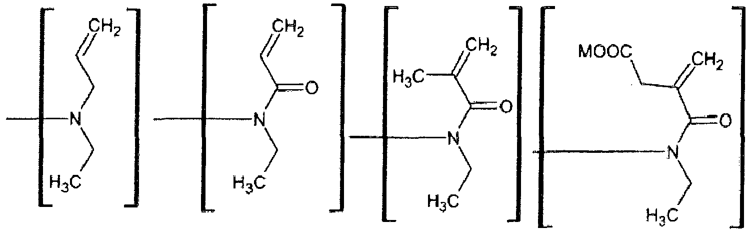

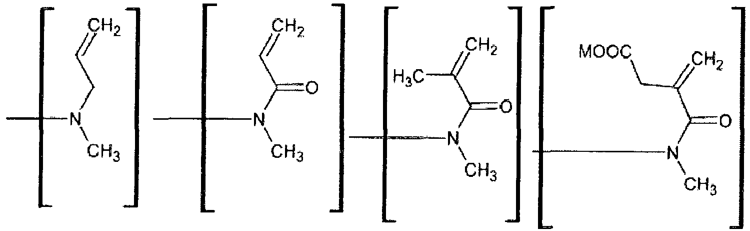

When B is a group of the formula (V), B is as follows.

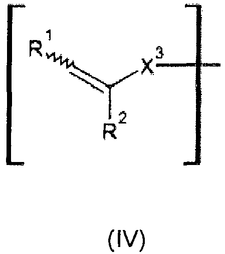

In formula (V), X³ is CH₂, CO, -CH₂CO-, CS, or -CH₂CS-,

According to a preferred embodiment, X³ is CO, -CH₂CO-.

In formula (V), R¹ and R² are independent from each other and independently have the same meaning as defined for formula (II). Moreover, in case more than one group R¹ and R², respectively, is present when more than one group B of formula (IV) is present in the polymerizable compound of formula (I), each of the groups R¹ and R² may be the same or different, respectively.

Specifically, when B is a group according to formula (V), R¹ is a hydrogen atom, -COOM, a straight chain or branched C₁₋₁₆ alkyl group which may be s ubstituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or -SO₃M.

According to a preferred embodiment when B is a group according to formula (V), R¹ is a hydrogen atom. In case R¹ is a C₁₋₁₆ alkyl group, the C₁₋₁₆ alkyl group is preferably substituted by -COOM.

When B is a group according to formula (V), R² is a hydrogen atom, -COOM, a straight chain or branched C₁₋₁₆ alkyl group which may be substituted by a C₆₋₁₄ aryl or C₃₋₁₄ heteroaryl group, -COOM, -PO₃M, -O-PO₃M₂ and -SO₃M, a C₃₋₆ cycloalkyl group which may be substituted by a C₁₋₁₆ alkyl group, a C₆₋₁₄ aryl or C₃₋₁₄ heteroaryl group, -COOM, - PO₃M, -O-PO₃M₂ or -SO₃M, or a C₆₋₁₄ aryl group which may be substituted by -COOM, - PO₃M, -O-PO₃M₂ and -SO₃M,

According to a preferred embodiment of B being a group according to formula (V), R² is a hydrogen atom or a methyl group.

Preferred groups of formula (V) are as follows, whereby M is a hydrogen atom or a metal atom.

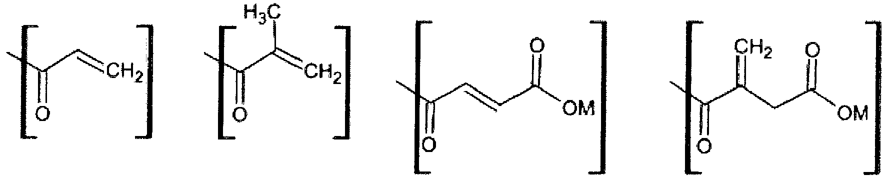

When B is a group [Z'X"]ₘE, the meaning of Z', X", m, and E is as follows.

Z' is a straight chain or branched C₁₋₄ alkylene group. Specific examples of the C₁₋₄ alkylene groups are methylene, ethylene, propylene and butylene.

X" is an oxygen atom, a sulfur atom or NR, (wherein R is a hydrogen atom, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, - COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or -SO₃M).

E is a hydrogen atom, PO₃M₂, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, -PO₃M, -O-PO₃M₂ or - SO₃M.

m is an integer of from 1 to 10.

In formula (I), n is an integer of from from 1 to 6, preferably an integer of from 1 to 3. According to a first preferred generic group of compounds of formula (I), n is 1. According to a second preferred generic group of compounds of formula (I), n is 2.

According to the definition of the present invention, the M which are independent from each other, each represent a hydrogen atom or a metal atom. The metal atom is preferably an alkali metal, an alkaline earth metal, or zinc. Specific examples of the alkali metal are lithium, sodium, and potassium. Specific examples of the alkaline earth metal are calcium, magnesium and zinc.

The polymerizable compound of formula (I) may be prepared according to the following scheme, wherein A represent polymerizable moieties, B and C represent moieties which may form a linkage B-C. The linkage B-C may be formed by a condensation reaction or an addition reaction. The reaction may involve the formation of an amide bond, a urethane bond, and an ester bond. Preferably, the reaction involves the formation of an amide bond or a urethane bond.

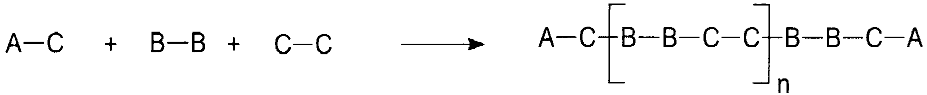

The preparation reaction may be carried out in a mixture of a compound having a single moiety C, a compound having two moieties B and a compound having two moieties C. Accordingly, a chain distribution of (B-B-C-C)n is observed whereby the certain positions such as the terminal positions, may be capped by a moiety A. Alternatively, it is possible to prepare a polymerizable compound of formula (I) by a reaction wherein a stoichiometric mixture of compound A-C and B-B is reacted and then the reaction product is reacted with a compound C-C. Alternatively, it is possible to prepare a polymerizable compound of formula (I) by a reaction wherein a mixture of compound B-B and C-C is reacted and then the reaction product is end-capped with A-C.

A specific example is as follows.

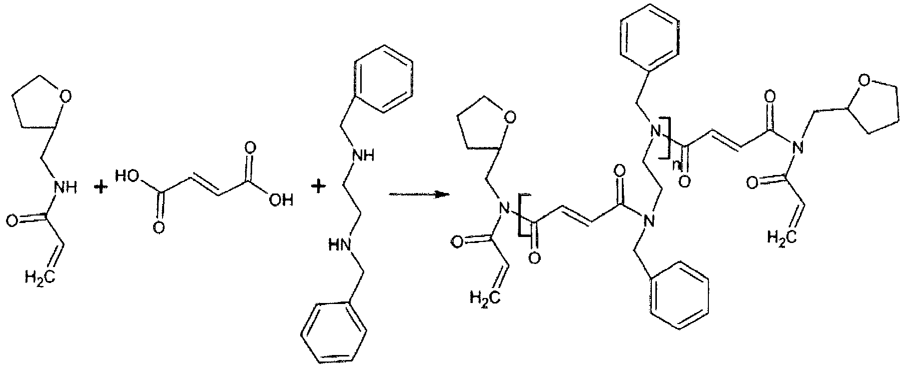

The reaction may be performed in accordance with, for example, the methods described in Jerry March "Advanced Organic Chemistry" Fourth Edition, John Wiley & Sons, INC., 1992 Richard C. Larock "Comprehensive Organic Transformation", VCH Publishers, INC., 1989 or a method conforming to the methods described above. The reaction may be carried out in a solvent which is capable of dissolving the reactants. An example of a suitable solvent is toluene. The reaction tempeature is not particularly limited. A suitable reaction temperature is from -30 °C to the boiling point of the solvent. The reaction time is not particularly limited and may be selected from 5 minutes to 48 hours.

### Initiator

The dental composition according to the present invention may comprise a polymerization initiator system. The initiator system may be based on a redox initiator or on a photoinitiator.

In case the dental composition contains a redox initiator, the amount of reducing agent and oxidizing agent should be sufficient to provide the desired degree of polymerization. Preferably, the mixed but unset cements of the invention contain a combined weight of about 0.01 to about 10%, more preferably about 0.2 to about 5%, and most preferably about 0.3 to about 3% of the reducing agent and oxidizing agent, based on the total weight (including water) of the mixed but unset cement components. The reducing agent or the oxidizing agent can be microencapsulated as described in U.S. Pat. No. 5,154,762. This will generally enhance shelf stability of the cement parts and if necessary permit packaging both the reducing agent and oxidizing agent together. Water-soluble and water-insoluble encapsulants can be employed. Suitable encapsulating materials include cellulosic materials as cellulose acetate, cellulose acetate butyrate, ethyl cellulose, hydroxymethyl cellulose and hydroxyethyl cellulose being preferred. Other encapsulants include polystyrene, copolymers of polystyrene with other vinylic monomers and polymethylmethacrylate, copolymers of methylmethacrylate with other ethylenically-unsaturated monomers. Preferred encapsulants are ethylcellulose and cellulose acetate butyrate. By varying the choice of encapsulant and the encapsulation conditions, the onset of curing can be tailored to start at times ranging from seconds to minutes. The ratio of amount of encapsulant to activator generally ranges from 0.5 to about 10 and preferably from about 2 to about 6.

Suitable oxidizing agents (initiators) include peroxides such as benzoyl peroxide cumene hydroperoxide (CHP), and tert-butyl hydroperoxide, ferric chloride, hydroxylamine (depending upon the choice of reducing agent), NO releasing agents, perboric acid and its salts, and salts of a permanganate or persulfate anion. Preferred oxidizing agents are peroxides, potassium persulfate, ammonium persulfate and hydrogen peroxide.

Suitable reducing agents (activators) include ascorbic acid, benzyl thiourea ferrous chloride, ferrous sulfate, hydrazine, hydroxylamine (depending upon the choice of oxidizing agent) oxalic acid, thiourea, and salts of a dithionite or sulfite anion. Preferred reducing agents include ascorbic acid and ferrous sulfate.

A photoinitiator should be capable of promoting polymerization of the polymerizable groups on exposure to light of a suitable wavelength and intensity. The photoinitiator preferably is sufficiently shelf-stable and free of undesirable coloration to permit its storage and use under typical dental conditions. Visible light photoinitiators are preferred. Suitable visible light-induced and ultraviolet light-induced initiators include an alpha-diketone (e.g., camphorquinone) with or without additional hydrogen donors (such as sodium benzene sulfinate, amines and amine alcohols). The photoinitiator may be present in an amount sufficient to provide the desired rate of photopolymerization. This amount will be dependent in part on the light source, the thickness of the cement layer to be exposed to radiant energy and the extinction coefficient of the photoinitiator. Preferably, mixed but unset photocurable cements of the invention will contain about 0.01 to about 5%, more preferably from about 0.1 to about 2% photoinitiator, based on the total weight (including water) of the mixed but unset cement components.

The invention will now be further explained based on the following examples:

### Example 1

### N-allyl-N-tetrahydrofurfurylacrylamide

4.95 g of tetrahydrofurfuryl bromide (30 mmol) were stirred under cooling. 8.56 g of allylamine (150 mmol, 5.00 eq) were added under cooling. The resulting mixture was stirred for 24 hours at room temperature. The excess of allylamine was then distilled off (bp. 53°C). The residue was resolved in 20 ml of dichloromethane and stirred. 4.53 g of acryloyl chloride (50 mmol, 1.6 eq) were added dropwise under cooling. Afterwards, 8.10 g of triethylamine (80 mmol) were added dropwise, also under cooling. The resulting mixture was stirred for 24 hours at room temperature. Then the solution was extracted three times with 100 ml of water. The organic layer was dried over sodium sulfate and concentrated under reduced pressure.

The obtained oily crude product was then purified by vacuum distillation (bp. 60-63°C, 2.7 x 10⁻² mbar). Yield: 66 %, η = 8.57 mPa*s (23°C),
**¹H-NMR** (600 MHz, CDCl₃): δ[*ppm*] = 1.46-1.59 (*m*, 1H), 1.87 (*ddq,* 2H, *J* = 26.9, 12.3, 7.3, 6.2 Hz, 8,8'-H), 1.95-2.04 (*m*, 1H), 3.11 (*dd,* 1H, *J* = 13.9, 7.7 Hz, 6-H), 3.33-3.47 (*m*, 1H, 6'-H), 3.69-3.78 (*m*, 1H, 7-H ?), 3.84 (*td,* 2H, *J* = 10.6, 4.6 Hz, 3,3'-H), 4.07-4.22 (*m*, 2H, 10,10'-H), 5.08-5.22 (*m*, 2H, 5,5'-H), 5.65 (dd, 1H, *J* = 10.4, 2.0 Hz, 1'-H), 5.74-5.89 (*m*, 1H, 4-H), 6.30-6.38 (*m*, 1H, 1-H), 6.49 (*dd*, 1H, *J* = 16.7, 10.4 Hz, 2-H).

### Example 2

### 1,3-Bis[acryloyl (tetrahydrofuran-2-yl) methyl amino]propane-2-ol

### A) 1,3-Bis[(tetrahydrofuran-2-yl) methyl amino] propane-2-ol

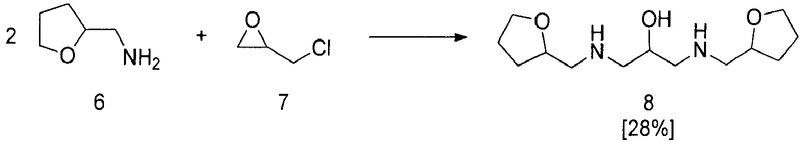

A 250 mL three-necked flask, equipped with septum and condenser, was charged with 26.3 mL 2-tetrahydrofurfurylamine (255 mmol) solved in 40 mL ethanol and was heated to 90 °C (oil bath temperature). Then, with continuous stirring, 5 mL epichlorohydrin (64 mmol) was added dropwise over four hours and the mixture was stirred further for 16 hours under reflux. After that, 50 mL 1 N HCl was added and the product was extracted with dichloromethane three times. The combined organics were dried over MgSO₄ and evaporated under vacuum to dryness to give a red-brown liquid. 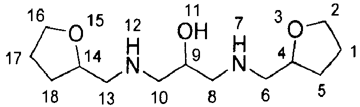
- **Yield:**: 4.67 g (28 % d.Th.)
- **IR v [cm⁻¹]:**: 3314 (m, br), 2969 (w), 2932 (w), 2867 (s), 1637 (m), 1452 (s), 1359 (w), 1284 (m), 1183 (w), 1058 (s), 918 (m), 876 (m), 810 (m), 732 (m)
- **¹H-NMR [ppm]:**: (300 MHz, CDCl₃): 1.47-1.59 (m, 2H, **H 5, 18),** 1.80-2.01 (m, 6H, **H 1, 5, 17, 18),** 2.58-2.80 (m, 8H, H **6, 8, 10, 13),** 2.98 (s, 2H, **H 7, 12),** 3.69-3.87 (m, 5H, **H 2, 9, 16),** 3.94-4.04 (m, 2H, **H 4, 14)**

### B) 1,3-Bis[acryloyl (tetrahydrofuran-2-yl) methyl amino] propane-2-ol

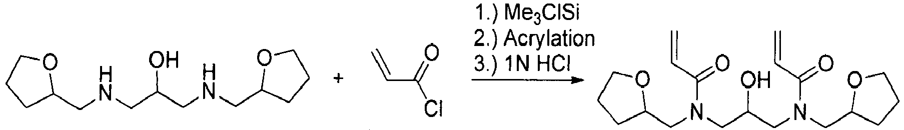

A 500 mL three-necked flask, equipped with septum and condenser, was charged with 5.78 g 1,3-Bis(((tetrahydrofuran-2-yl)methyl)amino)propan-2-ol (22.4 mmol) solved in 200 mL dichloromethane and 31.0 mL triethylamine (223.7 mmol) under nitrogene atmosphere. The mixture was cooled to 0 °C and 10.0 mL chlorotrimethylsilane (78.3 mmol) was added dropwise. After complete addition the reaction stirred overnight. Then, 9.0 mL acryloyl chloride (111.9 mmol) solved in 50 mL dichloromethane was added dropwise keeping the temperature at -78 °C and stirred at room temperature for about 4 hours. After that, 150 mL 1 N HCl was added and stirred for 2 hours. The organic layer was washed with 100 mL 1 N HCl three times, dried over MgSO₄, filtered and evaporated under vacuum to dryness. The residue was purified by flash column chromatography eluting with CH₂Cl₂/EtOH (volume ratio, 95:5) to give a light yellow liquid.

Due to the fact that the product polymerized during high vacuum drying the analytical results are based on the only slightly dried product. 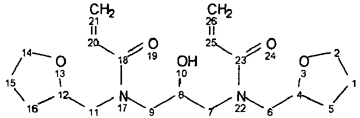
- **Yield:**: 2.28 g (28 % d.Th.),
- **IR v [cm⁻¹]:**: 3354 (s, br), 2963 (m), 2933 (m), 2875 (m), 1641 (s), 1603 (s), 1587 (s), 1444 (s), 1427 (s), 1370 (m), 1271 (w), 1230 (s), 1133 (m), 1097 (m), 1059 (m), 977 (m), 895 (w), 794 (s), 745 (w), 639 (w)
- **¹H-NMR [ppm]:**: (300 MHz, CDCl₃): 1.40-1.53 (m, **2H, H 5a, 16a),** 1.82-2.06 (m, 6 H, **H 1, 15, 5b, 16b),** 2.87-4.37 (m, 15 H, H **2, 4, 6, 7, 8, 9, 11, 12, 14),** 5.66 (d, 2H, ³J_{H}= 10.3 Hz, **H 21a, 26a),** 6.32 (d, 2H, ³J_{H}= 16.2 Hz, H 21b, 26b), 6.62 (dd, 2H, ³J_{H}= 16.4 Hz, ³J_{H}= 10.4 Hz, **H 20, 25)**
- **MS (ESI):**: m/z = 367.22 [M+H]

## Claims

1. Dental composition comprising a polymerizable compound of the following formula (I):
A-L(B)ₙ (I)
wherein
A is a group of the following formula (II) 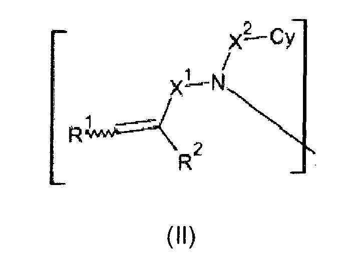
X¹ is CO or CS;
X² is a single bond, CO, CS, CH₂, or a group Z[X'Z']ₖ, wherein X' is an oxygen atom, a sulfur atom or NR, (wherein R is a hydrogen atom, a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -O-PO₃M₂ or -SO₃M, a C₆₋₁₄ aryl or C₃₋₁₄ heteroaryl group which may be substituted by -COOM, - PO₃M, -O-PO₃M₂ or -SO₃M), Z is a straight chain or branched C₁₋₄ alkylene group, Z' is a bond, a straight chain or branched C₁₋₄ alkylene group, and k is an integer of from 1 to 50;
Cy is a monocylic, bicyclic or tricyclic cycloalkyl group having 3 to 20 carbon atoms or a monocylic, bicyclic or tricyclic heterocycloalkyl group having 2 to 20 carbon atoms and 1 to 4 heteroatoms selected from oxygen, sulfur and nitrogen atoms, which groups may be substituted with 1 to 3 groups selected from a C₁₋₁₆ alkyl group, - COOM, -PO₃M, -O-PO₃M₂ or -SO₃M;
R¹ is a hydrogen atom,
-COOM,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -0-PO₃M₂ or -SO₃M,
a C₆₋₁₄ aryl or C₃₋₁₄ heteroaryl group which may be substituted by - COOM, -PO₃M, -O-PO₃M₂ or -SO₃M,
R² is a hydrogen atom,
-COOM
a straight chain or branched C₁₋₁₆ alkyl group which may be substituted by a C₆₋₁₄ aryl or C₃₋₁₄ heteroaryl group, -COOM, -PO₃M, -O-PO₃M₂ and -SO₃M,
a C₃₋₆ cycloalkyl group which may be substituted by a C₁₋₁₆ alkyl group, a C₆₋₁₄ aryl or C₃₋₁₄ heteroaryl group, -COOM, -PO₃M, -0-PO₃M₂ or -SO₃M, or
a C₆₋₁₄ aryl or C₃₋₁₄ heteroaryl group which may be substituted by - COOM, -PO₃M, -O-PO₃M₂ and -SO₃M,
L is a single bond or an (n+1) -valent linker group;
B independently is
a group according to the definition of A,
a group of the following formula (III) 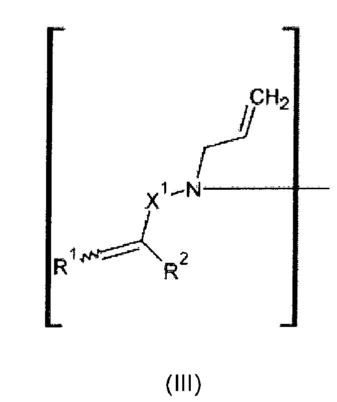 wherein
X¹ independently has the same meaning as defined for X¹ in formula (II), CH₂, or a group [X'Z]_{k'}, wherein X' is an oxygen atom, a sulfur atom or NH, Z is a straight chain or branched C₁₋₄ alkylene group, and k' is an integer of from 1 to 50;
R¹ and R² are independent from each other and independently have the same meaning as defined for formula (II),
a group of the following formula (IV) 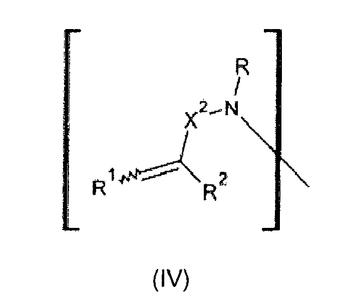 wherein
X² independently has the same meaning as defined for X¹ in formula (III),
R¹ and R² are independent from each other and independently have the same meaning as defined for formula (II),
R is a hydrogen atom,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, - PO₃M, -O-PO₃M₂ or -SO₃M,
a C₆₋₁₄ aryl or C₃₋₁₄ heteroaryl group which may be substituted by -COOM, -PO₃M, -O-PO₃M₂ or -SO₃M,
a group of the following formula (V) 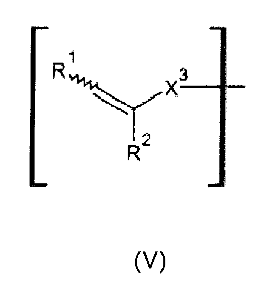 wherein
X³ is CH₂, CO, -CH₂CO-, CS, or -CH₂CS-,
R¹ and R² which are independent from each other and independently have the same meaning as defined for formula (II), or
a group [Z'X"]ₘE,
wherein
Z' is a straight chain or branched C₁₋₄ alkylene group,
X" is an oxygen atom, a sulfur atom or NR, (wherein R is a hydrogen atom, a straight chain or branched C₁₋₁₆ alkyl group which may be substituted by a C₃₋₆ cycloalkyl group, a C₆₋₁₄ aryl or C₃₋₁₄ heteroaryl group, -COOM, - PO₃M, -O-PO₃M₂ or -SO₃M, a C₃₋₆ cycloalkyl group which may be substituted by a C₁₋₁₆ alkyl group, a C₆₋₁₄ aryl or C₃₋₁₄ heteroaryl group, -COOM, -PO₃M, -0-PO₃M₂ or -SO₃M, a C₆₋₁₄ aryl or C₃₋₁₄ heteroaryl group which may be substituted by -COOM, -PO₃M, -0-PO₃M₂ or -SO₃M),
E is a hydrogen atom,
PO₃M₂,
a straight chain or branched C₁₋₁₆ alkyl group which may be substituted by a C₃₋₆ cycloalkyl group, a C₆₋₁₄ aryl or C₃₋₁₄ heteroaryl group, -COOM, -PO₃M, -0-PO₃M₂ or -SO₃M,
a C₃₋₆ cycloalkyl group which may be substituted by a C₁₋₁₆ alkyl group, a C₆₋₁₄ aryl or C₃₋₁₄ heteroaryl group, - COOM, -PO₃M, -O-PO₃M₂ or -SO₃M,
a C₆₋₁₄ aryl or C₃₋₁₄ heteroaryl group which may be substituted by -COOM, -PO₃M, -O-PO₃M₂ or -SO₃M, and
m is an integer of from 1 to 10; and
n is an integer of from from 1 to 6;
wherein M which are independent from each other each represent a hydrogen atom or a metal atom;
provided that in case L is a single bond, B cannot be a group according to the definition of A or a group of the formula (III) or (IV).

2. The dental composition according to claim 1, wherein Cy is selected from the following groups:

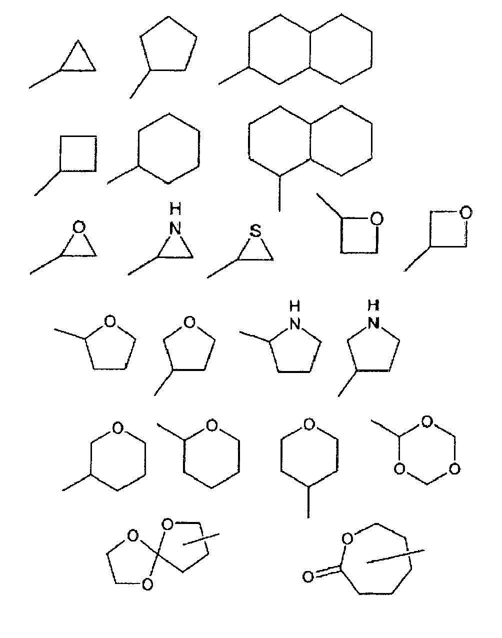

3. The dental composition according to claim 1 or 2, wherein n is 1.

4. The dental composition according to claim 1 or 2, wherein L is a single bond.

5. The dental composition according to claim 1 or 2, wherein L is a linker group.

6. The dental composition according to any one of the preceding claims wherein X¹ is CO.

7. The dental composition according to any one of claims 1, 2, 3 or 5, wherein the linker group of L is a C₁₋₁₆ hydrocarbon group which may contain 1 to 3 carbonyl groups or heteroatoms selected from oxygen, nitrogen and sulfur, and which may be substituted by a hydroxyl group, a C₆₋₁₄ aryl group, -COOM, -PO₃M, -O-PO₃M₂ or - SO₃M, wherein M is a hydrogen atom or a metal atom.

8. The dental composition according to claim 7, wherein X¹ is CO.

9. The dental composition according to claim 7, wherein R¹ is a hydrogen atom and R² is a hydrogen atom or a methyl group.

10. The dental composition according to claim 5, wherein the linker group is a polyamide residue of the following formula (VI)
-Y-L'-Y'- (VI)
wherein
Y and Y' represent CO and the polyamide moiety of formula (VI) is obtainable by a process comprising the step of a step-growth polymerization of a mixture containing a compound having at least two amino groups of the following formula (VII) and a compound of the following formula (VIII) having at least two carboxylic acid groups,
R³(NHR")_{y} (VII)
wherein
R³ represents an y-valent C₂₋₂₀ straight-chain, branched or cyclic hydrocarbon group which may optionally contain from 1 to 6 hetero atoms selected from nitrogen, oxygen, or sulphur atoms in the backbone of the hydrocarbon group, and optionally from 1 to 6 functional groups selected from hydroxyl groups, thiol groups and amino groups;
R" represents a hydrogen atom, an allyl group a straight chain or branched C₁₋₆ alkyl group which may be substituted by a C₃₋₆ cycloalkyl group, or a C₆₋₁₄ aryl group, a C₃₋₆ cycloalkyl group which may be substituted by a C₁₋₆ alkyl group or a C₆₋₁₄ aryl group; and
y represents an integer of at least 2;
MOOC-R⁴-COOM (VIII)
wherein R⁴ represents a C₁₋₂₀ straight-chain, branched, cyclic or aromatic hydrocarbon group which may optionally contain a carbon-carbon double bond, from 1 to 6 heteroatoms selected from nitrogen, oxygen, or sulphur atoms in the backbone of the hydrocarbon group, and optionally from 1 to 6 functional groups selected from carboxylic acid groups, hydroxyl groups, thiol groups and amino groups, and the M which may be the same or different, independently represent a hydrogen atom or a metal atom.

11. The dental composition according to any one of the preceding claims, wherein at least one of X¹ and Y is CO.

12. The dental composition according to any one of the preceding claims, wherein the compound of formula (I) has an average molecular weight of from 100 to 10,000, and/or which is selected from a dental adhesive composition, a bonding agent, a pit and fissure sealant, a dental desensitizing composition, a pulp capping composition, a dental composite, dental glass ionomer cement, resin modified glass ionomer, a dental cement and a dental root canal sealer composition.

13. The dental composition according to any one of the claims 1 or 4, wherein B is not a group of formula (II), (III) or (IV).

14. Use of a compound of formula (I) as defined in claim 1, for the preparation of a dental composition selected from a dental adhesive composition, a bonding agent, a pit and fissure sealant, a dental desensitizing composition, a pulp capping composition, a dental composite, dental glass ionomer cement, resin modified glass ionomer, a dental cement and a dental root canal sealer composition.
